# EUROPEAN PATENT APPLICATION

(11) **EP 4 173 658 A1**
(43) Date of publication of application: **03.05.2023**
(21) Application number: 21204736.9
(22) Date of filing: 26.10.2021
(51) Int. Cl.: A61M 5/315

(54) **MAGNETIC FIELD SENSOR FOR A DRUG DELIVERY DEVICE**

(71) Applicant: Ypsomed AG, 3401 Burgdorf (CH)
(72) Inventor: Schrul, Christian, 3400 Burgdorf (CH); Scheurer, Simon, 3006 Bern (CH); Bosshard, Simon Martin, 3006 Bern (CH); Brügger, Martin, 3065 Bolligen (CH)
(74) Representative: Meier Obertüfer, Jürg

(57) **Abstract**

The invention relates to a drug delivery arrangement comprising a drug delivery device (1) for dispensing a drug from a reservoir (3), the device (1) comprising a device housing (4) defining an axial direction, a movable dispensing member (40), a magnetic element (21) with a permanent magnetization coupled to or integrally formed in the dispensing member (40). The arrangement includes further an electronic module (50) for monitoring a dispensing process executed by the drug delivery device (1) and releasably attachable to the device housing (4), the module (50) comprises a module body, a magnetic field sensor unit (52a-52c) and a controller, wherein the sensor unit (52a-52c) is adapted to measure an absolute rotational position and an absolute axial position of the magnetic element (21) and wherein the controller is configured to determine on basis of both the absolute rotational and the absolute axial position a fill level or a total amount of dispensed drug.

## Description

### FIELD OF THE INVENTION

The present invention relates to injection devices or medicament delivery devices for injecting, delivering, administering, infusing or dispensing substances and/or liquids such as insulin, hormone preparations or vaccines. It departs from a drug delivery arrangement including a drug delivery device and an electronic module releasably attachable to a delivery device. The electronic module comprises a magnetic field sensor unit adapted to sense a magnetic element in the drug delivery device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from an electronic module or electronic unit embedded or integrated in the delivery device, or being part of an auxiliary or supplemental electronic module or add-on device releasably attachable to the delivery device. The electronic module monitors a drug delivery process, in order to proactively prevent false handling of the device and/or to keep track of the doses already applied, and generates data related to an instantaneous condition and/or use of the delivery device.

For example, WO 2016/142216 A1 discloses an injection device and an electronic module releasably attachable to the injection device. The injection device comprises an elongated member axially displaceable inside a device housing. A magnetization of adjacently arranged sections of the elongated member changes by a constant angle of 90°. The spiral or helical-shaped magnetization of the elongated member is similar to the one of a Hallbach array. The electronic module comprises a magnetic field sensor including a longitudinal row of individual magnetic field sensor elements. By means of the sensor elements an actual axial position of the elongated member is detectable.

WO 2017/013464 A1 discloses a dose control system with an injection device comprising a dose selector. On a dose selector shaft a magnet is mounted which is rotatable and axially movable together with the dose selector. Changes in the magnetic field and polarity are detected by a magnetometer arranged in an add-on attached at proximal end of the injection device. The variations in magnetic field can be resolved by a central processing unit in the add-on and therefrom an angular position of rotation is calculated.

WO 2019/110494 A1 discloses a drug delivery device comprising a generally ring-formed magnet integrally with a reset tube. The magnet serves as an indicator rotating during expelling of a dose amount. The amount of rotational movement is indicative of the size of an expelled dose amount. An add-on comprises sensor electronics and is configured to sample data from magnetometers. This allows a rotational start position of the magnet to be determined prior to release of the expelling mechanism.

These prior art approaches suffer from the fact that an axial sensing requires a comparatively large attachable module or in case of rotational sensing the number of rotations has to be counted by an electronic circuit. If the user detaches the electronic module or if the sensing between the attached module and the rotatable member in delivery device is interrupted the rotational member has to be brought in a reset or start condition as the electronic circuit has lost the number of counts and hence the absolute position.

### DESCRIPTION OF THE INVENTION

It is an objective of the invention to provide a reliable sensing in a space saving design of a drug delivery process by means of an electronic module attachable to the drug delivery device.

This objective is achieved by the drug delivery arrangement, the electronic module and the method according to the independent claims. Preferred embodiments are evident from the dependent claims.

The invention relates to a drug delivery arrangement comprising a drug delivery device for dispensing a drug from a reservoir, preferably included in the delivery device. The delivery device comprises a device housing defining an axial or longitudinal direction and preferably comprising a housing extending in the axial or longitudinal direction. The delivery device further includes a movable dispensing member, wherein a position of the dispensing member relative to the delivery device housing unambiguously represents or is indicative of a fill level of the drug in the reservoir. The delivery device includes further a magnetic element with a permanent magnetization and a spatial magnetic field, wherein the magnetic element is operatively coupled (rotationally and translationally) to the dispensing member or integrally formed (as a monobloc) in the dispensing member.

The drug delivery arrangement further includes an electronic module for monitoring a dispensing process executed or performed by the drug delivery device. The electronic module is releasably attachable (non-destructive detachable and reusable) to the device housing. The module comprises a module body, a magnetic field sensor unit (including at least one magnetic field sensor) and a controller electrically connected to the sensor unit. The sensor unit is adapted to measure an absolute rotational position of the magnetic element relative to the device housing by sensing at least one magnetic field component of the spatial magnetic field of the magnetic element. The sensor unit is further configured to measure an absolute axial position of the magnetic element relative to the device housing and the controller is configured to determine on basis of both the measured absolute rotational and the measured absolute axial position a fill level or a total amount of dispensed drug. The latter includes the amount of drug that has been dispensed since a dispensing from a full reservoir has started. The fill level means the amount of drug currently remaining in the reservoir.

According to the invention the sensor measures both the absolute rotational position and the absolute axial position. In contrast to prior art approaches the position is not determined based on counted relative movement or cumulated moving steps, e. g. counting rotations.

The measured absolute rotational position in combination with the measured absolute axial position provide a unique and unambiguous position information. That means the position of the magnetic element and thus the position of the dispensing member relative to the device housing can be reliably determined at any time. By way of example, if the user detaches the electronic module during dispensing or in-between two subsequent administrations the electronic module is able to reliably recognize an instantaneous position of the dispensing member as soon as the electronic module is reattached to the device housing.

The absolute position measuring may be in particular advantageous if the dispensing member travels only a very short distance from a full level to an end level when the reservoir is empty. In this case the axial position measuring is probably not sufficiently accurate. As the dispensing member moves preferably in rotational direction as well as in axial direction the measured rotational position adds further position information to the axial position information.

In prior art approaches that measure exclusively an axial displacement the several magnetic elements have to be arranged next to each other in axial direction and thus demand for a relatively long construction. As according to the present invention the absolute rotational and absolute axial position are measured at the same time the magnetic elements can be arranged in a space saving design.

On the other hand, a sole measurement of the rotational position of the dispensing member may be ambiguous and the axial position information provides an unambiguous indication where the dispensing member is positioned relative to the device housing or the module body.

The dispensing member (for example a plunger rod, drive sleeve, clutch) is part of the drug dispensing mechanism. The dispensing member is movable relative to the device housing, preferably in a dispensing direction to move an element inside the reservoir towards an opening in the reservoir in order to eject or expel the drug through the opening out of the reservoir. Thus, a specific movement of the dispensing member causes a specific amount of drug to be expelled. That means the dispensing member is movable along a travel path for the drug dispensing. For example, when the dispensing member is in an initial or start position the reservoir is full and when the dispensing member is at the end of its travel path the reservoir is empty. Each position of the dispensing member is thus assigned to a specific fill level and thus each possible position of the dispensing member unambiguously represents a fill level of the drug in the reservoir.

The magnetic field sensor unit includes at least one magnetic field sensor adapted to sense a magnetic field or/and a change in a magnetic field. The sensor unit may have directivities in different directions or planes preferably orthogonally arranged to each other.

The sensor unit is preferably adapted to measure at least two magnetic field components of the spatial magnetic field of the magnetic element. A first field component may represent the rotational displacement or position and a second field component may represent an axial translation or position of the magnetic element. Hence, the sensor unit is adapted to measure in two directions or planes preferably orthogonally arranged to each other. Each measuring direction or plane may have its output at the sensor unit.

As a measured position of the dispensing member (and thus a measured position of the magnetic element as the latter is coupled to the dispensing member) represents a fill level of the drug in the reservoir the controller is able to determine an instantaneous fill level or an already dispensed amount of dose with respect to a total capacity of drug in the reservoir.

The already dispensed amount of drug can be calculated, for example, by subtracting the determined fill level (remaining drug) from the total capacity of the reservoir or the fill level can be calculated by subtracting a measured already dispensed amount of drug from the total capacity of the reservoir.

The controller may have access to reservoir information data. Examples for such information are scale matching the travel path of the dispensing member to different fill levels or a look-up table matching discrete dispensing member positions to discrete fill levels. These information and/or information about a total capacity of the reservoir may be, for example, previously stored in the electronic module or may be received via a data transmission, for example, from a readable data tag on the reservoir or from an external sender via wireless data communication.

As mentioned above the dispensing member is an element of the dispensing mechanism of the delivery device and is movable relative to the device housing. The movement may be a rotational, a translational, a screw movement or a combination thereof.

The magnetic element is either operatively coupled to the dispensing member such that a movement of the dispensing member is transmitted to the magnetic element. The term coupled means the magnetic element can be directly or indirectly coupled to the dispensing member via one or more intermediate member. The transmission of the movement between the dispensing member and the magnetic element may be translated by means of a gear. The magnetic element may be only rotational, only axially or both rotationally and axially coupled, connected or fixed to the dispensing member.

Alternatively, the magnetic element may be integrated in the dispensing member. In this case the magnetic element and the dispensing member are monolithic, for example, if the dispensing member is injection molded onto the magnetic element or the dispensing member is made of a magnetized material or magnetic material such as a magnetic metal or a plastic magnet (for example PANiCNQ).

The drug delivery device may be an injection device, preferably a disposable injection device, such as an injection pen or a patch injector applicable onto the skin of the user for the duration of the injection. Alternatively, the drug delivery device may be an infusion system, for example, a conventional medical drug pump such as an insulin pump with tubing or it may be a drug patch pump without tubing that is attachable directly onto the skin of the user.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

The electronic module is releasably attachable to the device housing, for example, by a clamping mechanism or a releasable snap-fit connection. Such modules are also named as supplementary device or add-on. The module is a separate device. Although it may interact with the delivery device the module does not comprise any drug or medication. The electronic module preferably comprises an electronic circuit including the controller. The latter may be a microcontroller, a digital processing unit or the like.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

In a preferred embodiment the dispensing member is a plunger rod rotatable and axially movable relative to the device housing. Preferably, the plunger rod is rotated and axially moved at the same time and thus screwed in dispensing direction. If the magnetic element is connected to the plunger rod this may provide a space saving design as the magnetic element may be arranged, for example, at a distal end of the plunger rod.

The magnetic element is operatively coupled to the plunger rod, preferably rotationally fixed relative to the plunger rod. Additionally, the magnetic element may be axially fixed to the plunger rod or it may be axially moveable relative to the plunger rod. In the latter case the axial moving component of the magnetic element may be different than the axial moving component of the plunger rod relative to the housing during a dispensing movement. This may be achieved, for example, if the plunger rod and the magnetic element are both in threaded connection with the housing but the plunger rod thread has a different ratio than the magnetic element thread in the housing.

In an alternative embodiment the magnetic element may be integrated in the plunger rod, for example, by magnetic particles, magnetized material or the plunger rod is made of magnetic material.

Preferably, the magnetic element is integrated in or part of a disc-shaped or rod-shaped member rotationally connected to the dispensing member. The disc-shaped form allows a space saving construction. Alternatively, the magnetic element may be cuboid-shaped with axial and/or diametric magnetization.

The disc-shaped member includes preferably alternatingly magnetized magnetic elements from magnetic north pole to magnetic south pole in circumferential direction (along a periphery of the member). In this way a rotational position or a rotational movement of the disc-shaped magnetic element is easy and reliably to detect by the sensor unit.

Preferably, the magnetic elements are equally distributed in the circumferential direction. In this embodiment the magnetic poles or pole angles may be equally distributed. For example, the magnetization may be directed radially and a corresponding south pole or north pole may be present on a radial outside of the magnetic element. Alternatively, the magnetization may be in form of a Hallbach array causing the magnetic field on the radial outside to be augmented while cancelling the field to near zero on a radial inner side.

The rotational axis of the disc-shaped member is preferably coaxially arranged to the rotational axis of the dispensing member. The disc-shaped member may include a plurality of circumferentially arranged magnetic elements with poles, for example, distributed such that every 36° a north or south pole is arranged. The disc-shaped member may comprise a plurality of poles, preferably between 2 and 40 poles.

In a preferred embodiment the disc-shaped member further includes at least two concentrically arranged ring-shaped areas, wherein each area includes a set of the above mentioned alternatingly magnetized elements. That allows to arrange a plurality of magnetic poles in a compact design. The disc-shaped member may include two to four concentrically arranged magnetic ring elements.

In a preferred embodiment the drug delivery device comprises a dose member for setting a dose and the delivery device additionally comprises a second magnetic element coupled to or integrally formed in the dose member. The above mentioned sensor unit is preferably a first sensor unit and the electronic module includes in this embodiment a second sensor unit adapted to sense an absolute rotational and absolute axial position of the second magnetic element and wherein the controller is configured to determine a set dose on basis of both the rotational and axial position of the second magnetic element relative to the housing. Hence, the device comprises the first magnetic element coupled to the dispensing member and a second magnetic element coupled to the dose member.

The second sensor unit can be implemented in a second electronic module housing separated from a first electronic module housing that includes the first sensor unit. In this case the first and second electronic module housing are both attachable to the housing of the drug delivery device. Alternatively, the second sensor unit may be implemented in the same module housing that includes the first sensor unit. In this alternative embodiment the module housing has an elongated form in order to be able to cover or reach the first magnetic element of the dispensing member and the second magnetic element of the dose member.

The dose member can preferably be screwed out of the housing and the second sensor unit is adapted to precisely determine the rotational and axial position or a movement of the dose member. The position of the dose member relative to the device housing unambiguously represents a set dose (dialed dose) and therefore the controller can determine a dose set by the user. The controller may have access to dose member information such as a scale or look-up table matching specific dose member positions to specific dose values.

As the controller can determine the remaining dose amount, the fill level or the dose amount already dispensed on basis of the position of the first magnetic element the controller is configured to determine both the position of the dispensing member and the position of the dose member relative to the device housing. That enables the controller to compare a set dose with a remaining dose. These determine positions may be stored in the electronic module or the position information may be wirelessly sent via a communication unit to an external receiver.

In a preferred embodiment the controller is configured to provide a notification to the user if the determined remaining dose amount determined on basis of the fill level is smaller than the determined set dose.

The controller can notify the user, for example, via user interface on the electronic module (display, LEDs, acoustic output) or via user computer or mobile device in case a set dose exceeds the remaining dose. This leads to a split dose meaning that the user has to split the set dose into two partial doses. One part of the dose has to be administered with the current reservoir and the other part with another reservoir. In case the delivery device is a fully disposable device the user has to take a new delivery device for the second part. In case the device is reusable or semi-disposable device the user has to replace the reservoir or the disposable part of the device including the drug.

The dose setting member is preferably a dose sleeve and the magnetic element is integrated in an outer wall of the dose sleeve. This arrangement of the magnetic elements allows an easy sensing of the magnetic field and hence a reliable position detection by the second sensor unit. The second magnetic element may be a cupped or shell-like element inserted in an outer wall of the dose sleeve such that its magnetic field can be easily detected by the second sensor unit.

The dose sleeve further includes preferably a third magnetic element axially spaced apart from the second magnetic element wherein the third magnetic element is integrated in the wall too. The axial arrangement of the second and third magnetic element may be advantageous for sensing the axial position or an axial displacement of the dose sleeve relative to the device housing in particular to provide a magnetic field of sufficient strength for the sensors in the axial direction.

Advantageously, the sensor unit comprises a Hall effect sensor adapted to sense a magnetic field in three dimensions. That means the sensor unit is able to detect magnetic field components in three different axis, which are orthogonal to each other. For example, a magnetic field can be sensed in an x, y and z direction. The sensor can thus sense linear as well as angular movements of the magnetic element. The sensor unit may include two or more individual magnetic field sensors to sense at least two different dimensions.

Alternatively, the sensor unit includes other types of magnetic field sensors, such as reed contact sensors.

In a preferred embodiment further comprises a data communication unit connected to the controller and adapted to communicate wirelessly with an external device. The communication unit allows to receive data from an external device or the controller may send data to the external device. The latter may be, for example, a user device, a cloud server or a computer of a HCP, a health facilities or a drug or device manufacturer. By way of example, the controller may receive a user therapy plan including a dose amount to be dispensed. Accordingly, the controller can verify on basis of the determined dispensing member position whether the remaining dose is sufficient for the planed dose amount according to the therapy plan.

Furthermore, the electronic module may, for example, receive user instruction or settings via communication unit and the controller may send recorded dispensing data to the external device.

In a preferred embodiment the electronic module includes an engaging member movable relative to the module body and adapted to engage the dispensing member or a dose member of the drug delivery device such that one of the engaging member and the dispensing or dose member causes a movement of the other of the dispensing or dose member and the engaging member.

That means a movement of the engaging member is physically transferred to the dispensing or dose member when the electronic module is attached to the drug delivery device. Alternatively, a movement of the dispensing or dose member may transferred to the engaging member in the electronic module. Therefore, the electronic module can control or interact with the dose or dispensing member via engaging member.

The engaging member is preferably an arm, hook, lever or protrusion protruding out of a housing of the electronic module. The delivery device comprises an opening or hole in its outer housing such that the engaging member can be inserted into the opening when the electronic module is attached to the delivery device. In the attached state the engaging member engages the dispensing or dose member, for example, by teeth, hook or by a contact surface.

For example, the engaging member may be a toothed wheel and the dispensing member may include corresponding teeth such that a rotation of the engaging member may cause a (dispensing) rotation of the dispensing member. In another example embodiment the engaging member may be a stop arm adapted to linearly move into the delivery device and to limit an axial movement of the dose member and thus limit the dose setting range. This may prevent, for example, the user from setting a dose that is not in accordance with a therapy plan.

The drug delivery device is preferably an injection device and more preferably a pen-shaped injection device. Furthermore, the injection device is preferably a fully disposable injection device. That means the device is discarded after use or if the reservoir is empty and the drug is completely dispensed. Disposable injection devices can be manufactured on low cost as the device does not have to comprise parts that can be disassembled and reused by the user.

Alternatively, the injection device may be a reusable or semi-disposable injection device. A semi-disposable injection device comprises components or sub-assemblies that are disposed of (disposable parts forming a disposable unit) after an injection or once the drug is fully dispensed. Additionally, it comprises further components or sub-assemblies that can be reused (reusable part) for several injections and successively with several identical disposable parts.

The invention relates further to the electronic module for monitoring a dispensing process of a drug from a reservoir with a drug delivery device and releasably attachable to the drug delivery device, the module comprising a module body, a magnetic field sensor unit and a controller connected to the sensor unit, wherein the sensor unit is adapted to measure an absolute rotational position of a magnetic element of the delivery device relative to the module body. A position of the magnetic element relative to the module body represents a fill level of the drug in the reservoir. The sensor unit is further configured to measure an absolute axial position of the magnetic element relative to the module body and wherein the controller is configured to determine on basis of both the absolute rotational and the absolute axial position a dispensing condition, in particular a fill level or a total amount of dispensed drug.

The invention further relates to a method of monitoring a dispensing process of a drug from a reservoir (3) with a drug delivery device (1) by means of an electronic module (50) releasably attached to the drug delivery device (1), the method comprising the steps of
a. measuring, by a magnetic field sensor unit (52a-52c) of the electronic module, a magnetic field of a movable magnetic element (21) in the drug delivery device (1);
b. determining, by a controller of the electronic module (50), an absolute rotational position and an absolute axial position of the magnetic element (21) relative to a delivery device housing (4) on basis of the measured magnetic field, wherein a position of the magnetic element (21) relative to the device housing represents a fill level of the drug in the reservoir (3);
c. calculating, by the controller, on basis of both the determined absolute rotational and axial position, a fill level or a total amount of dispensed drug.

A drug delivery arrangement not according to the claims may comprise a drug delivery device for dispensing a drug from a reservoir, wherein the drug delivery device includes
a device housing defining an axial direction;
a first rotatable dispensing member rotating during a dose dispensing;
a first magnetic element coupled to or integrally formed in the first dispensing member;
an electronic module for monitoring a dispensing process of the drug delivery device and releasably attachable to the drug delivery device, the module comprising a first magnetic field sensor and a controller and wherein the first sensor is adapted to measure an absolute rotational position of the first magnetic element relative to the device housing.

The drug delivery device further comprises a second rotatable dispensing member different from the first dispensing member and rotating during dose dispensing, and a second magnetic element coupled to or integrally formed in the second dispensing member, wherein the electronic module comprises a second magnetic field sensor adapted to measure an absolute rotational position of the second magnetic element relative to the device housing wherein a combination of the position of the first magnetic element with the position of the second magnetic element represents an unambiguous fill level of the drug in the reservoir and wherein the controller is configured to calculate on basis of the determined combination a fill level or a total amount of dispensed drug.

Non limiting examples of the first and second dispensing member are toothed wheels, spur wheels, unit wheels, dose setting sleeves or drive sleeves of the dispensing mechanism of the delivery device.

Preferably, the first sensor has a directivity in a first plane and the second sensor has a directivity in a second plane, wherein the first plane is parallel to and axially spaced apart from the second plane.

Preferably, the drug delivery device further comprises a third rotatable dispensing member different from the first and second dispensing member and rotating during dose dispensing, and a third magnetic element coupled to or integrally formed in the third dispensing member and wherein the electronic module comprises a third magnetic field sensor adapted to measure an absolute rotational position of the third magnetic element relative to the device housing. The position of the third magnetic element may further specify an instantaneous fill level of drug in the reservoir.

Preferably, the first and second dispensing member are operatively coupled to each other. If any, preferably the third dispensing member is operatively coupled to the second dispensing member.

In a preferred embodiment the first dispensing member is a first unit wheel and the second dispensing member is a second unit wheel of a dose counter mechanism to indicate the amount of drug dispensed from or remaining in the reservoir, wherein the first and second unit wheel are arranged next to each other. If any, a third dispensing member is preferably a third unit wheel arranged next to the second or first unit wheel.

Preferably, the second unit wheel is coupled to the first unit wheel by means of a gear which translates a continuous rotation movement of the first unit wheel into an intermittent rotation of the second unit wheel.

Preferably, the first unit wheel and the second unit wheel (and if any the third unit wheel) have a peripheral surface onto which markings are arranged, preferably in the form of the numbers 0 to 9. Hence, preferably, the first unit wheel is indicative of the amount of medicament dispensed with or remaining in the drug delivery device or a cartridge connected therewith. Further preferably, the second unit wheel is indicative of every tenth unit or decade of units, such as like 10, 20, 30, and 40 and so on, preferably up to 90, of the amount of medicament dispensed with or remaining in the drug delivery device or a cartridge connected therewith. Hence, the gear which translates the continuous rotation of the first unit wheel into an intermittent rotation of the second unit wheel is preferably configured such that the second unit wheel is rotated about one tenth of a full rotation for every full rotation of the first unit wheel. In a preferred embodiment, the gear may be in the form of a Geneva mechanism.

The dose counter preferably includes a third unit wheel which is arranged next to the second unit wheel and which is coupled to the second unit wheel by means of a second gear which allows a partial rotation of the third unit wheel for every full rotation of the second unit wheel, said partial rotation preferably being a tenth of a full rotation.

The first unit wheel is preferably coupled to a plunger rod of the drug delivery such that a rotational movement of the plunger rod causes a rotational movement of first unit wheel. The first unit wheel may be directly coupled to the plunger rod or the first unit wheel may be indirectly coupled to the plunger rod, for example via one or more intermediate gear members.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an injection pen with an attached electronic module according to the invention;
- Fig. 2: depicts a sectional view of a middle portion in axial direction of the injection pen with the electronic module;
- Fig. 3: depicts a sectional view of the injection pen and the electronic module in a plan perpendicular to the axial direction;
- Fig. 4: depicts a schematic view of magnetic elements of a disc member of the electronic module;
- Fig. 5: depicts a an injection pen with an attached electronic module of a second embodiment;
- Fig. 6: depicts a sectional view of a middle portion of the second embodiment;
- Fig. 7: depicts a sectional view in a plane perpendicular to the axial direction of the second embodiment;
- Fig. 8: depicts the injection pen with an alternative electronic module of the second embodiment;
- Fig. 9: depicts an injection pen with an attached electronic module according to a third embodiment
- Fig. 10, 11: depict sectional views of the third embodiment;
- Fig. 12, 13: depict sectional views of a fourth embodiment;
- Fig. 14: depicts an injection pen with a dose counter mechanism and an electronic module;
- Fig. 15: depicts a detailed view of the dose counter mechanism and
- Fig. 16: depicts a schematic view of the arrangement of the magnetic elements in the dose counter mechanism and the magnetic field sensors.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

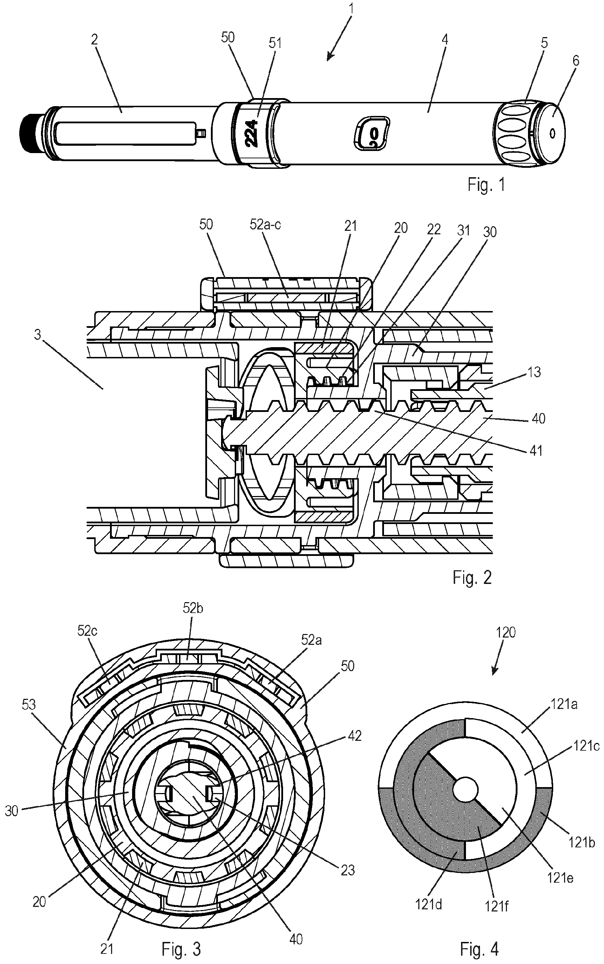

In the present description the term "distal" refers to the side where the needle is attached. This is on the left hand side in the figures 1, 2, 5, 8, 9, 11, 12, 14 and 15. The term "proximal" refers to the opposite side furthest away from the needle side (right hand side in the above indicated figures).

Figure 1 shows a perspective view of a drug delivery device according to the invention. The delivery device is implemented as a fully disposable injection pen 1. The injection pen 1 includes a manually operated dose and dispensing mechanism inside a pen housing 4, a reservoir holder 2 connected to the housing and adapted to holding a reservoir in form of a cartridge 3, a dose knob 5 and a dispensing button 6. On the housing 4 of the injection pen 1 an electronic module (add-on) 50 according to the invention is releasably attached. The electronic module 50 includes a magnetic field sensor unit as described in the following in detail and includes a display 51.

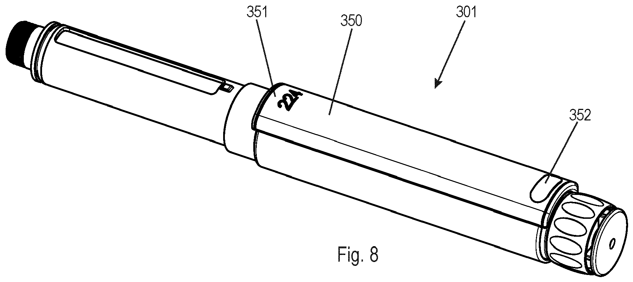

The dose and dispensing mechanism inside the housing 4 is descripted in detail in EP20216386.1, in particular with respect to figure 8 to 9b of the EP20216386.1. The document is incorporated herein by reference.

Figure 2 depicts a sectional view of a middle part of the injection pen 1 in an enlarged view and shows a distal portion of the dose and dispensing mechanism. A mechanics holder 30 is non-movable connected to the housing 4 and arranged therein. The mechanics holder 30 includes in its center a bore with an inner thread 31. A plunger rod 40 comprises an outer thread 41 and is in engagement with the inner thread 31 of a mechanics holder 30. During dose dispensing the plunger rod 40 is screwed in distal direction to move a piston (not shown) inside the cartridge 3 to dispense a dose. The plunger rod 40 is driven by a drive sleeve 13.

The plunger rod 40 further includes longitudinal nuts or grooves 42 running along the axial direction. A disc member 20 coaxially arranged with respect to the plunger rod 40 comprises a central bore with axial splines 23 protruding into the grooves 42 of the plunger rod 40 and thereby rotationally locking the disc member 20 to the plunger rod 40 while allowing axial shifting. A proximal portion of the bore of the disc member 20 has a larger diameter and includes an inner thread 22 which is in threaded engagement with an outer thread 31 arranged on a sleeve-shaped element of the mechanics holder 30. Due to this thread connection the disc member 20 can rotate relative to the mechanics holder 30 and hence the disc member 20 and the plunger rod 40 can be screwed in distal direction. The thread pitch for the disc member 20 can be designed independently from the thread pitch for plunger rod 40. Preferably, the axial displacement of the disc member 20 is shorter than the axial displacement of the plunger rod 40 when both are screwed in dispensing direction.

The disc member 20 comprises ten magnetic elements 21 having a permanent magnetization. The magnetic elements 21 are equally distributed and circumferentially arranged in disc member 20, best shown in figure 3. As shown the magnetic elements 21 reach an outer surface of the disc member 20.

The magnetic elements 21 are circumferentially arranged and repeatedly alternate from north pole to south pole in circumferential direction. Alternatively, the magnetic elements 21 may be arranged to form a magnetic pattern such as a Hallbach array. Hence, the arrangement of the magnet elements 21 is such that the magnetic field strength may circumferentially vary in a defined pattern. That means the magnetic field strength in a specific rotational position can be unambiguously determined by magnetic field sensors 52a-52c of the attached electronic module 50.

The electronic module 50 is releasably hold on the housing 4 of the injection pen 1. For this purpose, the electronic module includes a ring structure 53 (figure 3) wherein the injection pen 1 can be axially inserted in an opening of the ring structure 53. The ring structure 53 includes a snapper (not shown) on its inside adapted to engage recesses or openings in the housing 4 of the injection pen 1 in order to provide a releasable connection. The snapper may be released if the user presses on an outside of the ring structure 53 and the electronic module 50 can be axially moved away. In the attached state the electronic module 50 is axially positioned in the range of the disc member 20.

The electronic module 50 includes a magnetic field sensor unit comprising three magnetic field sensors 52a-52c best shown in figure 3. This figure depicts a sectional view in a plane perpendicular to the axial direction of the injection pen 1. The magnetic field sensors 52a-52c are circumferentially arranged spaced apart in an upper portion of the electronic module 50. The sensors 52a-52c are connected to an electronic circuit (not shown) including a microcontroller. The sensors 52a-52c are three dimensional (3D) Hall effect sensors adapted to sense a magnetic field in three dimensions. That means the sensors 52a-52c can sense a magnetic field produced by the magnetic elements 21 of the disc member 20 in a rotational direction as well as in an axial direction.

Figure 4 shows a schematic view of an alternative embodiment of the disc member 120 including magnetic elements 121a-121f. The connection to the plunger rod is not shown in this schematic view but the connection may be implemented with groves and splines as described above.

According to the embodiment shown in figure 4 the magnetic elements 121a-121f are arranged with alternating polarity. The white colored sections represent a magnetic south pole while the grey sections represent a magnetic north pole. As shown the poles alternate in circumferential direction and additionally the disc member 120 includes in radial direction different coaxially arranged circles, wherein each circle has a different arrangement of the pole sections. This way, the magnetic elements 121a-121f produce a magnetic field that changes in circumferential directions and thus allows the sensor to unambiguously determine an absolute rotational position of the disc member 120.

Additionally, the electronic module 50 includes a storage module (not shown) connected to the microcontroller and a communication module (not shown) configured to send wirelessly data to an external device and configured to receive wirelessly data from the external device. The storage module includes a look-up table or a list with possible plunger rod positions (or disc member positions) assigned to corresponding fill levels of the drug in the cartridge 3. Hence, by means of the previously stored table or list the microcontroller is able to determine a fill level on basis of a measured plunger rod (or disc member) position. The table or list may be entered by the user, manufacturer depending on the cartridge used or the controller may receive the data via communication module from an external sender such as a user mobile phone, cloud server or computer of a HCP or device manufacturer.

The function of the disc member and sensing is described in the following with respect to figure 2. During dose dispensing the plunger rod 40 is driven by the drive sleeve 13 in a dispensing (distal) direction. That means the plunger rod 40 is set in rotation by the drive sleeve 13 and due to the thread 41 the plunger rod 40 is screwed in distal direction to dispense the drug from the cartridge 3. As the plunger rod 40 is rotationally splined to the disc member 20 the latter is rotated as well. However, the disc member 20 is in threaded engagement with the mechanics holder 30 via its inner thread 22 which thread has a smaller thread pitch than the thread 41 of the plunger rod 40. This causes the disc member 20 to be moved slower in distal direction than the plunger rod 40 meaning that the disc member 20 is displaced a shorter axial distance in distal direction than the plunger rod 40.

As the sensors 52a-52c are adapted to sense also in the axial direction the sensors 52a-52c can measure an absolute axial position of the disc member 20. Therefore, the microcontroller of the electronic module 50 is adapted to determine an absolute rotational and an absolute axial position of the disc member 20 on basis of the measured position of the magnetic elements 52a-52c. As each possible position of the disc member 20 and therefore a plunger rod position relative to the device housing 4 is assigned to a specific fill level of the drug inside the cartridge 3 the controller is configured to determine unambiguously a fill level of the drug or to determine a remaining amount of drug by subtracting the determined dispensed drug from a previously registered total amount of drug. The remaining amount of drug or a total amount of dispensed drug can be displayed to the user via display 51.

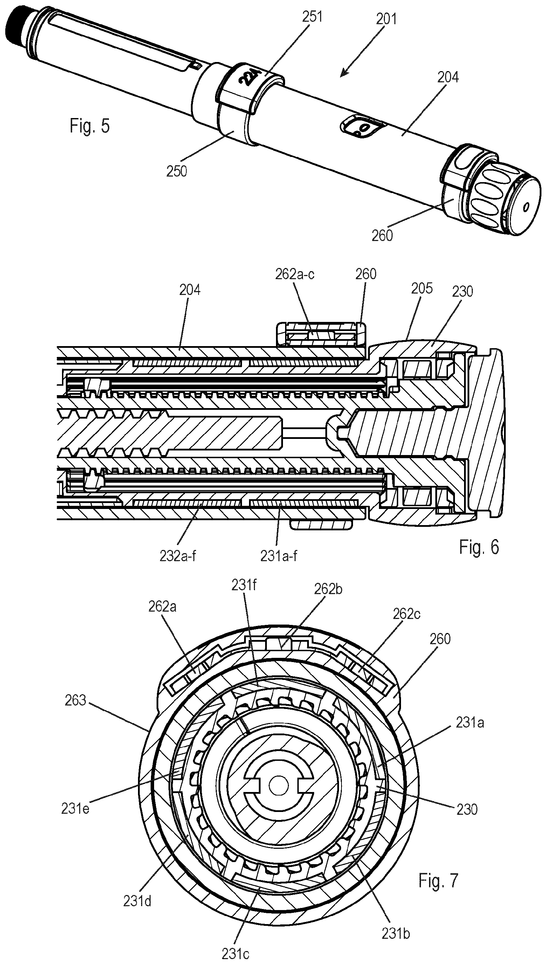

Figure 5 depicts a second embodiment of the present invention. The electronic module includes a first module housing that is attached to an injection pen 201. The injection pen includes a disc member with magnetic elements as described above. Accordingly, the releasably attachable first electronic module housing, named in this embodiment as dispensing module 250, includes a magnetic field sensor unit (first sensor unit) including magnetic field sensors positioned axially in the range of the disc member to sense an absolute rotational and axial position of the disc member and thus of the plunger rod as described above.

In addition the injection pen 201 according to the second embodiment shown in figures 5 to 7 includes magnetic elements 231a-f, 232a-f integrated in a dose sleeve 230 and a second electronic module housing (second sensor unit), named as dose setting module 260, axially positioned around the magnetic elements 231a-f, 232a-f of the dose sleeve 230 to sense a position of the dose sleeve 230 as described below. Thus, in the second embodiment the electronic module comprises a dispensing module 250 and a dose setting module 260. The microcontroller is not only able to sense a position of the plunger rod but additionally it is able to sense a position of the dose sleeve 230 by means of the dose setting module 260. The dose sleeve 230 is manually operated by the user.

As depicted in figure 6 a dose knob 205 is connected to the dose sleeve 230. The dose sleeve 230 is in threaded connection to a housing 204 and is screwed out of the housing 204 if a user rotates the dose knob 205 in order to set a dose. The dose sleeve 230 includes the magnetic elements 231a-f, 232a-f in an outer wall of the sleeve. As shown in figure 6 the magnetic elements 231a-f and 232a-f are arranged next to each other in axial direction. As best shown in figure 7 there are 6 magnetic elements 231a-f, 232a-f circumferentially positioned and equally distributed in circumferential direction. The magnetic elements 23 1a-f, 232a-f reach an outer contour or surface of the dose sleeve 230.

The magnetic elements 231a-f, 232a-f include different poles and are arranged such that a magnetic field strength changes in circumferential direction such that magnetic field sensors 262a-c in the dose setting module 260 are able to unambiguously determine a rotational and axial position of the dose sleeve 230.

As shown in figure 6 the dose setting module 260 is positioned axially in the range of the magnetic elements 231a-f, 232a-f and in a proximal portion of the injection pen 201. Similar to the first embodiment the dose setting module 260 comprises a ring structure 263 with a releasable clamping mechanism to hold the module 260 on the housing 204 of the injection pen 201.

The dose setting module 260 includes three magnetic field sensors 262a-262c in form of three dimensional Hall effect sensors as described above with respect to the first embodiment. Figure 7 depicts a sectional view in a plane perpendicular to the axial direction of the injection pen 201. As shown in figure 7 the three magnetic field sensors 262a-262c are arranged next to each other in circumferential direction. As the sensors 262a-262c can sense a magnetic field in three different dimensions the sensors can determine an absolute rotational as well as an absolute axial position of the dose sleeve 230. Hence, on basis of the sensor signals the microcontroller can determine a position of the dose sleeve 230 relative to the device housing 204 and thus the microcontroller is able to determine a dose set by user (dialed dose) as each possible position of the dose sleeve (axial and radial) is assigned to a specific dose. The determined set dose can be displayed to the user via display in the dose setting module 260.

The dose setting module 260 is wirelessly connected to the dispensing module 250 and data or signals generated by the magnetic field sensors in the two modules 250, 260 can be transmitted and interchanged between the modules 250, 260 by Bluetooth or NFC data transmission.

The microcontroller is preferably in the dispensing module 250 (alternatively in the dose setting module 260) and is able to determine an absolute position of the plunger rod 40 and thus of the fill level on basis of the magnetic field sensors 52a-52c (see above first embodiment) in the dispensing module 250. Furthermore, the microcontroller is further adapted to determine a set dose on basis of the determined position of the dose sleeve 230 on basis of the magnetic field sensors 262a-262c in the dose setting module 260. The microcontroller can further compare the dialed dose (the dose planned to be dispensed) and the remaining dose in the cartridge 3. If the dialed dose exceeds the remaining dose in the cartridge 3 the controller informs the user accordingly via display 251 of the dispensing module 250 (alternatively or additionally the dose setting module may include a display) and/or via user mobile device. That means the planned dose has to be split into two partial doses, one part with the current cartridge 3 or the current injection pen respectively and the other part with a new cartridge 3 or new injection pen. Such a notification is also named as split dose warning.

The dispensing module 250 (alternatively the dose setting module 260) can receive user specific therapy plan data via communication unit and the controller may inform the user upon receipt of the planed dose information if the dose amount according to the therapy plan exceeds the remaining dose amount.

Figure 8 depicts an alternative embodiment to the electronic module as shown in figure 5 to 7. As shown in figure 8 the electronic module 350 is attachable to the injection pen 301 and includes both the dispensing module (first sensor unit) and the dose setting module (second sensor unit) in one common module housing. For this purpose, the electronic module 350 is axially elongated and covers both the magnetic elements of the disc member and the magnetic elements of the dose sleeve. The module 350 further includes a dispensing display 351 adapted to display a remaining amount of dose and a dose setting display 352 to display a determined set dose. Furthermore, the electronic module 350 includes magnetic field sensors as described above. Structural and functional features of the magnetic sensing with the electronic module 350 correspond to the embodiment described above with respect to figures 5 to 7.

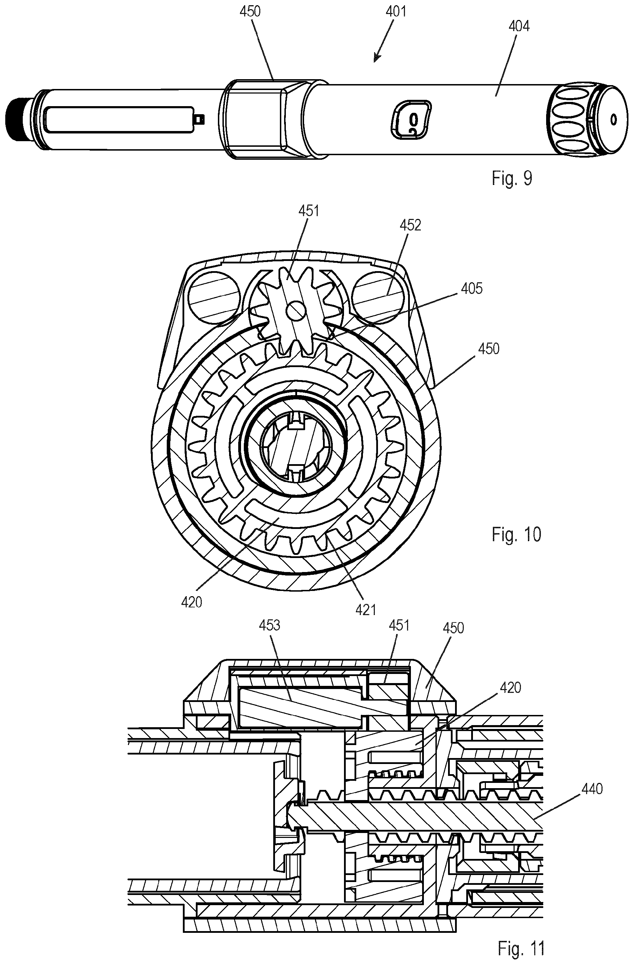

Figure 9 depicts a third embodiment of the present invention. As described with respect to the first embodiment (figures 1 to 4) the injection pen 401 includes a disc member 420 with magnetic elements (not shown) and an electronic module 450 releasable attached to the housing 404 of the injection pen 401 and including magnetic field sensors (not shown) to sense an absolute rotational and axial position of the disc member 420.

However, in contrast to the first embodiment the disc member 420 includes teeth 421 circumferentially arranged on an outside like a gear wheel. The housing 404 comprises an opening 405 (see figure 10) in a distal portion allowing access to the toothed disc member 420 from outside. The attached electronic dispensing module 450 includes a toothed drive wheel 451 engaging the teeth 421 of the disc member 420 through the opening in the housing 404. As best shown in figure 10 the toothed drive wheel 451 is driven by an electric motor 453 (figure 11) in form of a stepper motor inside the dispensing module 450 and controlled by a microcontroller of the module 450. Hence, the dispensing module 450 may drive the drive wheel 451 which causes the toothed disc member 420 in the injection pen 401 to rotate via gear. As the disc member 420 is rotationally coupled to the plunger rod 440 the latter is rotated too and thus screwed in distal direction. That means the dispensing module 450 can actively dispense a dose by means of the electric motor 453 and drive wheel 451 without uncouple the mechanism from a manual operating mode. The microcontroller in the module drives the motor 453 on basis of previously stored therapy plan data in the module 450 or received via communication unit. Such an automatic drive in an electronic module is described in EP 21176121.8 which is incorporated herein by reference.

All use cases and driving modes with the automatic drive in the electronic module disclosed in EP 21176121.8 can be combined with the magnetic field sensors as described herein with respect to the first embodiment (figures 1 to 4) and with the second embodiment (figures 5 to 8).

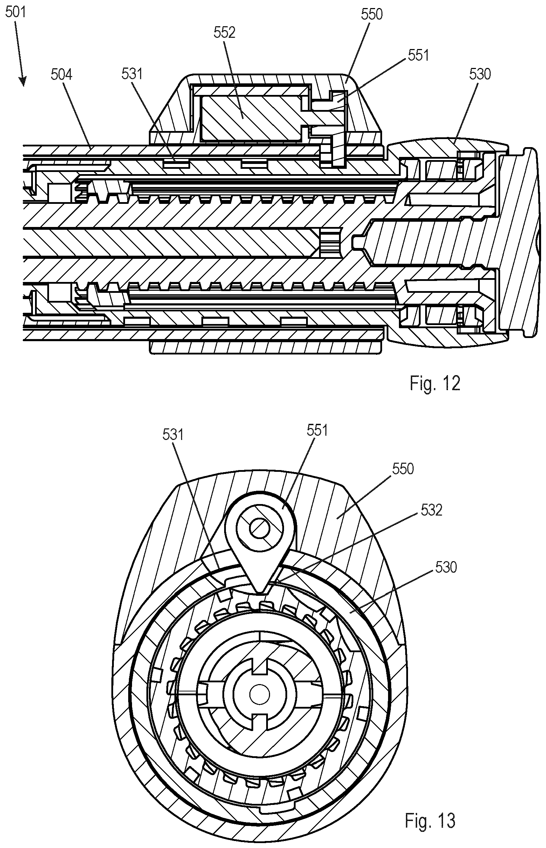

Figures 12 and 13 depict a forth embodiment of the present invention. The dispensing module 550 of the fourth embodiment includes all features described above with respect to the second embodiment (figures 5 to 7). Particularly, the injection pen 501 according to the fourth embodiment includes a dose sleeve 530 with magnetic elements (not shown) and an dose setting module 550 attached to a proximal portion of a housing 504 of the injection pen 501 and including magnetic field sensors (not shown) to sense an absolute rotational and axial position of the dose sleeve 530.

In addition to the dose sleeve of the second embodiment the dose sleeve 530 as shown in figure 12 includes on its outside helically arranged indentations 531 or depressions. Each indentation 531 includes a stop wall 532.

In addition to the features described with the second embodiment the dispensing module 550 according to the fourth embodiment shown in figures 12 and 13 includes a rotatable cam 551 (best shown in figure 13) adapted to engage with the indentation 531 and in particular with the stop wall 532 of the dose sleeve 530.

When a user rotates the dose sleeve 530 in order to set a dose the dose sleeve 530 screws out of the pen housing 504. The controller does not activate the cam 551 and therefore the cam 551 is pivotable. If the cam 551 is in the position shown in figure 12 and if the dose sleeve 530 is further rotated during dose setting the cam 551 is deflected or pivoted letting pass the stop wall 532 of an indentation 531 and thus allowing the dose sleeve 530 to be rotated.

The user can rotate the dose sleeve 530 and thus increase the dose as long as the electric motor 552 (figure 12) lets the cam 551 deflect. As the cam 551 is coupled to the motor 552 the controller is able to sense and count the number of cam deflections. Each deflection means that the user has increased the dose one unit. If a predefined number of maximum dose units or a predefined fixed dose is reached the controller activates the electric motor 552 which locks the cam 551 and prevents a pivoting or additional deflection of the cam 551. That means the user cannot rotate the dose sleeve 530 any further in the dosing direction as the cam 551 abuts the stop wall 532 and hence prevents a further rotation of the dose sleeve 530.

The controller in the dispensing module 550 may limit the movement of the dose sleeve 530 according to therapy plan data previously stored in the module 550 or received via communication unit from a cloud server or HCP. Detailed structural and functional features of such a dose limiting mechanism and possible uses cases are described in the above mentioned and incorporated patent application EP 21176121.8.

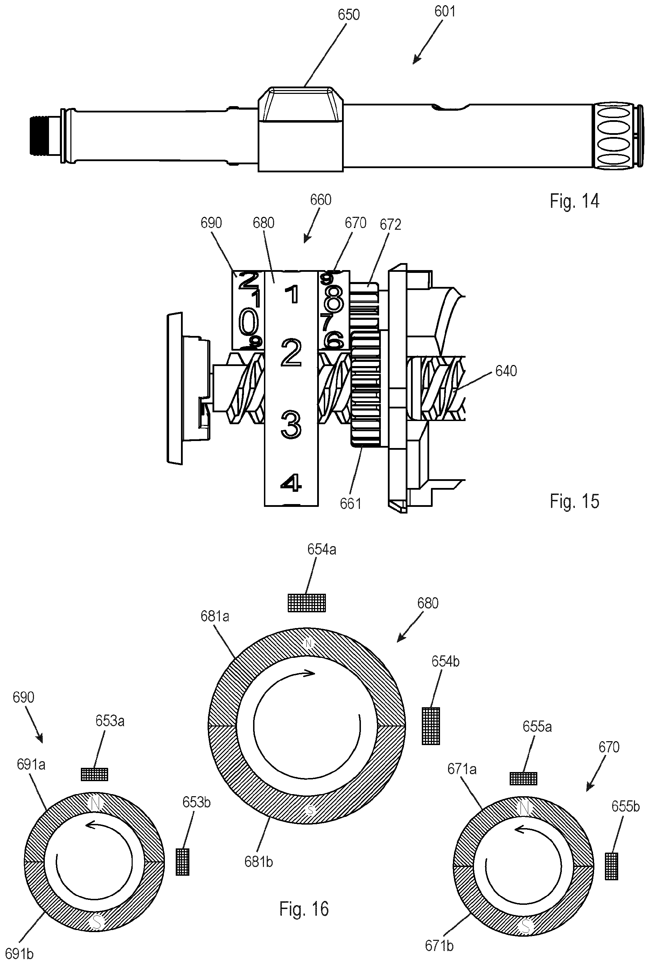

Figure 14 to 16 show an embodiment with injection pen 601 including a mechanical dose counter mechanism 660 and an electronic module 650 releasably attached to the injection pen 601. The dose counter mechanism 660 is shown in figure 15 in detail (without housing of injection pen) and includes a first unit wheel 670, a second unit wheel 680 and a third unit wheel 690 arranged next to each other along a central axis.

The first unit wheel 670 comprises a spur wheel section 672 on a proximal face. The spur wheel section 672 has teeth which mesh with a spur wheel 661 which is arranged concentric around the plunger rod 640. The spur wheel 661 is splined to the plunger rod such that it is rotationally coupled to the plunger rod 640 but axially shiftable relative to the plunger rod 640.

The second unit wheel 680 is coupled to the first unit wheel 670 by a gear in form of a first Geneva drive which translates a continuous rotation movement of the first unit wheel 670 into an intermittent rotation of the second unit wheel 680.

The third unit wheel 690 is arranged next to the second unit wheel 680 and is coupled to the second unit wheel 680 by means of a second Geneva drive which allows a partial rotation of the third unit wheel 690 for every full rotation of the second unit wheel 680. The partial rotation is a tenth of a full rotation.

The first, second and third unit wheel 670, 680, 690 each have a peripheral surface onto which markings are arranged in the form of the numbers 0 to 9. The second unit wheel is indicative of every tenth unit or decade of units, such as 10, 20, 30, 40 and up to 90. Similarly, the third unit wheel is indicative of every hundredth unit.

Hence, the first Geneva drive which translates the continuous rotation of the first unit wheel 670 into an intermittent rotation of the second unit wheel 680 is configured such that the second unit wheel 680 is rotated about one tenth of a full rotation for every full rotation of the first unit wheel 670 and the second Geneva drive is configured such that the third unit wheel 690 is rotated about one tenth of a full rotation for every full rotation of the second unit wheel 680.

The dose counter mechanism 660 is described in further detail in the patent application EP21178697.5 filed on 10 June 2021, which application is incorporated herein by reference.

Each unit wheel 670, 680, 690 includes two magnetic elements. Figure 16 depicts a schematic view of the arrangement of these magnetic elements and the magnetic field sensors of the attached electronic module 650. As shown in figure 16 the first unit wheel 670 includes a magnetic elements 671a having a magnetic north pole and a magnetic element 671b having a magnetic south pole. Similarly, the second unit wheel 680 includes two magnetic elements 681a, 681b and the third unit wheel 690 includes two magnetic elements 691a, 691b.

As shown in figure 14 the attached electronic module 650 is axially positioned in the middle of the length of the injection pen 601 and in the range of the dose counter mechanism 660. The electronic module 650 comprises in total 6 magnetic Hall effect sensors 653a,b, 654a,b and 655a,b.

As shown in figure 16 a first pair of magnetic field sensors 655a, 655b are circumferentially and 90° spaced apart from each other positioned and arranged outside of the first unit wheel 670 when the electronic module 650 is attached. Therefore, the sensors 655a, 655b are adapted to detect a rotational position of the first unit wheel 670 based on the magnetic field produced by the magnetic elements 671a, 671b.

Similarly, a second pair of magnetic field sensors 654a, 654b are positioned outside the second unit wheel 680 and are adapted to detect the position of the second unit wheel 680 by means of the magnetic elements 681a, 681b and a third pair of magnetic field sensors 653a, 653b are positioned outside the third unit wheel 690 and are adapted to detect the rotational position of the third unit wheel 690 by means of the magnetic elements 691a, 691b.

Hence, an absolute rotational position of each unit wheel 670, 680, 690 can be determined by the microcontroller in the electronic module 650. As each possible combination of rotational position of the unit wheels 670, 680, 690 unambiguously represents a specific plunger rod position a dispensed dose amount or a remaining dose can be determined by the microcontroller.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | injection pen | 401 | injection pen |
| 2 | reservoir holder | 404 | housing |
| 3 | reservoir | 405 | opening |
| 4 | housing | 420 | toothed disc member |
| 5 | dose knob | 421 | teeth |
| 6 | button | 440 | plunger rod |
| 20 | disc member | 450 | electronic dispensing module |
| 21 | magnetic elements | 451 | toothed drive wheel |
| 22 | inner thread | 452 | battery |
| 23 | spline | 453 | electric motor |
| 30 | mechanics holder | 501 | injection pen |
| 31 | thread | 504 | housing |
| 40 | plunger rod | 530 | dose sleeve |
| 41 | thread | 531 | indentations |
| 42 | longitudinal groove | 532 | stop wall |
| 50 | electronic module | 550 | dispensing module |
| 51 | display | 551 | cam |
| 52 | magnetic field sensor | 552 | electric motor |
| 53 | ring structure | 601 | injection pen |
| 120 | disc member | 650 | electronic module |
| 121a-f | magnetic elements | 653a,b | magnetic field sensors |
| 201 | injection pen | 654a,b | magnetic field sensors |
| 204 | housing | 655a,b | magnetic field sensors |
| 230 | dose sleeve | 640 | plunger rod |
| 231a-f | magnetic elements | 661 | spur wheel |
| 232a-f | magnetic elements | 670 | first unit wheel |
| 250 | dispensing module | 672 | spur wheel section |
| 251 | display | 671a,b | magnetic elements |
| 260 | dose setting module | 680 | second unit wheel |
| 262a-c | magnetic field sensors | 681a,b | magnetic elements |
| 263 | ring structure | 690 | third unit wheel |
| 301 | injection pen | 691a,b | magnetic elements |
| 350 | electronic module | | |
| 351 | dispensing display | | |
| 352 | dose setting display | | |

## Claims

1. A drug delivery arrangement comprising
• a drug delivery device (1) for dispensing a drug from a reservoir (3), the device (1) comprising
a device housing (4) defining an axial direction;
a movable dispensing member (40), wherein a position of the dispensing member (40) relative to the device housing (4) represents a fill level of the drug in the reservoir (3);
a magnetic element (21) with a permanent magnetization and a spatial magnetic field, coupled to or integrally formed in the dispensing member (40);
• an electronic module (50) for monitoring a dispensing process executed by the drug delivery device (1) and releasably attachable to the device housing (4), the module (50) comprising a magnetic field sensor unit (52a-52c) and a controller connected to the sensor unit,
wherein the sensor unit (52a-52c) is adapted to measure an absolute rotational position of the magnetic element (21) relative to the device housing (4)
**characterized in that**
the sensor unit (52a-52c) is further configured to measure an absolute axial position of the magnetic element (21) relative to the device housing (4) and wherein the controller is configured to determine on basis of both the absolute rotational and the absolute axial position the fill level or a total amount of dispensed drug.

2. Drug delivery system according to claim 1, wherein the dispensing member (40) is a plunger rod rotatable and axially movable relative to the device housing (4).

3. Drug delivery system according to claim 1 or 2, wherein the delivery device (1) includes a disc-shaped or rod-shaped member (20, 120) rotationally connected to the dispensing member (40) and comprising the magnetic element (21).

4. Drug delivery system according to claim 3 wherein the disc-shaped member (20, 120) includes alternatingly magnetized magnetic elements (21, 121a-121f) arranged along a periphery of the member (20, 120).

5. Drug delivery system according to claim 4 wherein the disc-shaped member (120) includes at least two concentrically arranged ring-shaped areas, wherein each ring-shaped area includes a set of alternatingly magnetized magnetic elements (121a-121f).

6. Drug delivery system according to any of claims 1 to 5 wherein the drug delivery device (201) comprises a dose member (230) for setting a dose and a second magnetic element (231a-f) coupled to or integrally formed in the dose member (230) and wherein the electronic module (250) includes a second sensor unit (262a-262c) adapted to sense an absolute rotational and an absolute axial position of the second magnetic element (231a-f) and wherein the controller is configured to determine a set dose based on both rotational and axial position of the second magnetic element (231a-f).

7. Drug delivery system according to claim 6, wherein the controller is configured to provide a notification to the user if a remaining dose amount determined on basis of the fill level is smaller than the determined set dose.

8. Drug delivery system according to claim 6 or 7 wherein the dose member (230) is a dose sleeve and wherein the magnetic element (231a-f) is integrated in a wall of the sleeve.

9. Drug delivery system according to claim 8 wherein the dose member (230) includes a third magnetic element (232a-f) axially spaced apart from the second magnetic element (231a-f).

10. Drug delivery system according to any of claims 1 to 9 wherein the sensor unit comprises (52a-52c) a Hall effect sensor adapted to sense a magnetic field in three dimensions.

11. Drug delivery system according to any of claims 1 to 10 wherein the electronic module (50) further comprises a data communication unit connected to the controller and adapted to communicate wirelessly with an external device.

12. Drug delivery system according to any of claims 1 to 11 wherein the electronic module (450, 550) includes an engaging member (451) movable relative to a module body of the electronic module (450, 550) and adapted to engage the dispensing member (440) of the drug delivery device (450) such that one of the engaging member (451) and the dispensing member (440) causes a movement of the other of the dispensing member (440) and the engaging member (451).

13. Drug delivery system according to any of claims 1 to 12 wherein the delivery device (1) is an injection device, preferably a disposable injection pen.

14. An electronic module (50) for monitoring a dispensing process of a drug from a reservoir (3) of a drug delivery device (1) and releasably attachable to the drug delivery device (1), the module (50) comprising a module body, a magnetic field sensor unit (52a-52c) and a controller connected to the sensor unit,
wherein the sensor unit (52a-52c) is adapted to measure an absolute rotational position of a magnetic element (21) of the delivery device (1) relative to the module body, wherein a position of the magnetic element (21) relative to the module body represents a fill level of the drug in the reservoir (3),
**characterized in that**
the sensor unit (52a-52c) is further configured to measure an absolute axial position of the magnetic element (21) relative to the module body and wherein the controller is configured to determine on basis of both the absolute rotational and the absolute axial position a fill level or a total amount of dispensed drug.

15. A method of monitoring a dispensing process of a drug from a reservoir (3) with a drug delivery device (1) by means of an electronic module (50) releasably attached to the drug delivery device (1), the method comprising the steps of
a. measuring, by a magnetic field sensor unit (52a-52c) of the electronic module, a magnetic field of a movable magnetic element (21) in the drug delivery device (1);
b. determining, by a controller of the electronic module (50), an absolute rotational position and an absolute axial position of the magnetic element (21) relative to a delivery device housing (4) on basis of the measured magnetic field, wherein a position of the magnetic element (21) relative to the device housing represents a fill level of the drug in the reservoir (3);
c. calculating, by the controller, on basis of both the determined absolute rotational and axial position, a fill level or a total amount of dispensed drug.
